(19) Europäisches Patentamt
European Patent Office
Office européen des brevets

(11) Numéro de publication: **0 424 679 B1**

(12) **FASCICULE DE BREVET EUROPEEN**

(45) Date de publication du fascicule du brevet: **20.12.95**

(51) Int. Cl.[6]: **A23L 3/3571**, C11B 5/00, A61K 7/00, A61K 47/22

(21) Numéro de dépôt: **90118535.5**

(22) Date de dépôt: **27.09.90**

(54) **Protection d'un produit alimentaire, cosmétique ou pharmaceutique de l'oxydation**

(30) Priorité: **27.10.89 CH 3891/89**

(43) Date de publication de la demande:
**02.05.91 Bulletin 91/18**

(45) Mention de la délivrance du brevet:
**20.12.95 Bulletin 95/51**

(84) Etats contractants désignés:
**AT BE DE DK ES FR GB IT NL SE**

(56) Documents cités:
**WO-A-86/04503**
**US-A- 2 333 656**

**WORLD PATENTS INDEX LATEST Section Ch, Week 8136, 1981 Derwent PublicationsLtd., London, GB; Class C, AN 81-65586D**

**WORLD PATENTS INDEX LATEST Section Ch, Week 8138, 1981 Derwent PublicationsLtd., London, GB; Class D, AN 81-69047D**

**Hawley's Condensed Chemical Dictionary, 11th. ed., Van Nostrand Reinold Company, New York, p. 298**

(73) Titulaire: **SOCIETE DES PRODUITS NESTLE S.A.**
**Case postale 353**
**CH-1800 Vevey (CH)**

(72) Inventeur: **Bracco, Umberto**
**Ch. E. Couvreu 2**
**CH-1800 Vevey (CH)**
Inventeur: **Löliger, Jurg**
**Ch. de Pierre à Fleur 6**
**CH-1802 Corseaux (CH)**
Inventeur: **Saucy, Françoise**
**6, Rte. de Châtel-St.-Denis**
**CH-1807 Blonay (CH)**

**EP 0 424 679 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne un procédé de protection d'une matière grasse ou d'un produit alimentaire, cosmétique ou pharmaceutique contenant une matière grasse de l'oxydation ainsi que l'utilisation de coenzyme Q comme antioxydant dans un produit alimentaire, cosmétique ou pharmaceutique contenant une matière grasse.

On sait que la coenzyme Q (CoQ) ou ubiquinone, que l'on a isolé des lipides des mitochondries intervient dans les mécanismes de base de production d'énergie par la respiration, dans le transport des électrons dans les mitochondries et dans la phosphorylation oxydative. On connaît son activité antioxydante dans les milieux biologiques, par exemple selon Littaru et al, Fats and perspectives, Drugs exptl.clin.Res X (7), 491-496. Cependant, dans un environnement différent comme un produit alimentaire, cosmétique ou pharmaceutique contenant des lipides on pourrait s'attendre à ce que la forme oxydée, la quinone, n'ait pas d'activité antioxydante, puisque les quinones sont normalement considérées comme étant des produits de désactivation des antibxydants du type hydroquinone.

WPIL Section C, week 8136 1981, Class C, 81-65586D, Derwent Publ., London (GB) & SU-A-789063 décrit l'utilisation d'ubiquinone Q10 pour augmenter le taux de viabilité d'une matière biologique.

US-A-2333656 décrit une méthode pour empêcher ou retarder l'oxydation des matières alimentaires, particulièrement des huiles et des matières grasses, selon laquelle des quinones et/ou des benzoquinones, éventuellement, éventuellement en combinaison avec l'acide ascorbique, sont utilisées.

WO-A-86/04503 décrit des compositions pharmaceutiques comprenant de la coenzyme Q10 et éventuellement de l'acide ascorbique. La composition donne la possibilité d'augmenter l'absorption de la CoQ10.

Nous avons trouvé de manière tout à fait inattendue que l'ubiquinone sous sa forme quinone, exerçait une activité antioxydante notable dans les produits alimentaires, cosmétiques ou pharmaceutiques contenant des lipides, en particulier des huiles riches en acides gras polyinsaturés.

Le procédé selon l'invention est caractérisé par le fait que l'on incorpore à la matière grasse ou au produit alimentaire, cosmétique ou pharmaceutique 0,1 à 5% en poids de coenzyme Q, par rapport au poids de matière grasse.

Dans le contexte de l'invention, le terme "produit alimentaire" est à comprendre dans un sens large couvrant les produits destinés à l'alimentation humaine ou animale, pourvu qu'ils contiennent des lipides susceptibles à l'oxydation. Dans le même ordre d'idées, un produit cosmétique ou pharmaceutique est à comprendre dans un sens large, destiné à l'application cutanée ou à l'ingestion par voie orale, entérale ou parentérale, pourvu qu'un tel produit contienne des lipides susceptibles à l'oxydation.

Selon l'invention, on entend par CoQ un dérivé quinonique de formule

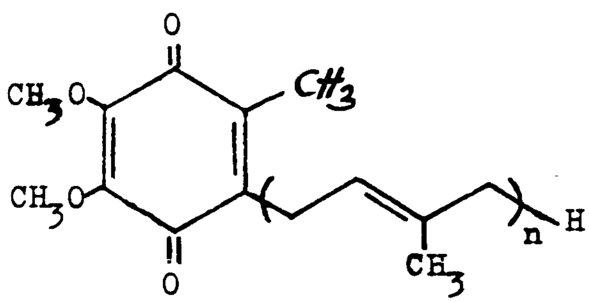

( I )

dans laquelle n = 6-10. Le composé $CoQ_{10}$, pour lequel n = 10 est préféré car il est le plus répandu et actuellement le seul disponible indutriellement.

Dans le présent procédé ou la présente utilisation, on incorpore la CoQ dans la phase lipidique de l'aliment, du produit cosmétique ou pharmaceutique à raison de 0,1 à 5% en poids par rapport aux lipides contenus dans un tel produit. Si l'on ajoute ou utilise moins de 0,1%, on risque de ne pas obtenir une protection appréciable des lipides du produit en question. Si l'on ajoute ou utilise plus de 5%, la protection obtenue n'est pas notablement plus grande que celle que l'on peut obtenir en ajoutant les quantités comprises dans le domaine indiqué.

Dans un mode de réalisation préféré du procédé selon l'invention, on utilise la CoQ, qui est liposoluble, en mélange avec d'autres antioxydants susceptibles de produire une synergie, par exemple l'acide ascorbique (AA), hydrosoluble, en présence d'un émulsifiant naturel.

Par "émulsifiant naturel" on entend selon l'invention les agents tensio-actifs présents dans la nature non ioniques, par exemple les saponines ou ioniques, par exemple les phospholipides, d'origine animale ou végétale, du lait, de l'oeuf, du soja, de préférence les lécithines, par exemple les lécithines commerciales, les lécithines purifiées, les fractions de lécithines de soja. La nature de l'émulsifiant utilisé n'a qu'une influence secondaire sur l'effet observé dans la mesure où il est capable de former une dispersion stable de l'AA dans un produit anhydre, par exemple une matière grasse ou un aliment contenant une matière grasse ou encore un produit cosmétique ou pharmaceutique contenant une matière grasse. On préfère utiliser les lécithines de soja ou leurs fractions qui sont abondantes et économiques.

Le mélange antioxydant préféré utilisable selon l'invention contient avantageusement 2,5 à 10%, de préférence environ 5% de CoQ et 2,5 à 20%, de préférence 5 à 20% d'AA, par rapport au poids d'émulsifiant naturel.

Bien entendu, on peut utiliser le mélange tel quel ou incorporer les différents composants du mélange de manière séparée à la matière grasse à protéger. Dans le cas, par exemple, où la matière grasse est une huile végétale contenant déjà naturellement de la lécithine (LC), par exemple l'huile de soja, il suffit d'ajouter la CoQ et l'AA en quantités efficaces.

Selon un mode de réalisation avantageux de la variante préférée ci-dessus, on prépare le mélange par mise en présence de la LC et de la CoQ sous agitation, à température inférieure ou égale à 60 °C, de préférence avec barbottage d'un gaz inerte, par exemple l'azote. A ce pré-mélange, on ajoute ensuite progressivement l'AA dissout dans un solvant polaire, de préférence à bas point d'ébullition, par exemple l'éthanol, puis on élimine le solvant à une température 60 °C, par exemple sous un léger vide. Le mélange obtenu se présente sous forme d'un liquide transparent et visqueux. Ce mélange peut être utilisé de manière différée, par exemple en l'incorporant dans une matière grasse à protéger, de préférence à chaud, le mélange étant à 60 °C environ, sous agitation vigoureuse.

Selon un autre mode de réalisation avantageux de la variante préférée ci-dessus, on incorpore l'AA et la CoQ à la matière grasse à laquelle on a préalablement ajouté la lécithine, de préférence sous forme d'une solution dans un solvant polaire, par exemple dans l'alcool éthylique, puis on élimine le solvant.

Les matières grasses à protéger selon l'invention sont de préférence les plus sensibles à l'oxydation, par exemple celles qui sont riches en acides gras insaturés, particulièrement polyinsaturés. On peut citer les huiles végétales, par exemple de germes de blé, de pépins de raisins, de maïs, de soja, de carthame, d'olive, d'onagre, de bourrache, spécialement l'huile de pépins de cassis. Comme matière grasse animale sensible à l'oxydation on peut citer la graisse de poule, l'huile de beurre, les huiles d'animaux marins, en particulier de poisson.

Les aliments, produits cosmétiques ou pharmaceutiques à protéger sont de préférence ceux qui contiennent de tels corps gras.

Les exemples suivants illustrent l'invention. Dans ceux-ci, les pourcentages et parties sont en poids, sauf indication contraire.

Exemple 1

Préparation des échantillons

On prépare des échantilons de 20 g d'huile stabilisée par addition de l'ubiquinone $CoQ_{10}$ (formule I, n = 10) à la quantité indiquée et mélange sous agitation, puis on dispose les échantillons dans des boîtes en fer étamé laquées stérilisées de 200 ml à raison de 0,5 g d'huile stabilisée d'un lot par boîte. On ferme les boîtes hermétiquement et on les entrepose à 37 °C.

Test d'oxydation accéléré

On analyse l'espace de tête des boîtes contenant les échantillons après un certain temps (en j) en déterminant la teneur en pentane et en éthane comme produits de la dégradation respectifs des acides linoléique et alpha-linolénique par l'oxydation, ainsi que la teneur en oxygène résiduel. A titre de comparaison, on effectue les mêmes analyses avec des échantillons sans antioxydant. On détermine les teneurs en pentane et en éthane par chromatographie en phase gazeuse et la teneur en oxygène par mesure de la susceptibilité paramagnétique.

Les résultats de ce test sont indiqués dans les tableaux 1 et 2 ci-après:

**Tableau 1**

| Durée d'entreposage (j) | 9 | | | 13 | | | 14 | | | 15 | | |
|---|---|---|---|---|---|---|---|---|---|---|---|---|
| Echantillon huile de poisson | Penthane M | Ethane M | 02 % | Pentane M | Ethane M | 02 % | Pentane M | Ethane M | 02 % | Pentane M | Ethane M | 02 % |
| avec 0,1% $CoQ_{10}$ | $9{,}55.10^{-12}$ | $7{,}99.10^{-11}$ | 14,7 | $9{,}10^{-10}$ | $1{,}6.10^{-8}$ | 0 | - | - | 0 | - | - | 0 |
| avec 1% $CoQ_{10}$ | $2{,}9.10^{-12}$ | $1{,}88.10^{-11}$ | 15,7 | $2{,}7.6^{-10}$ | $3{,}64.10^{-9}$ | 5,9 | $3{,}39.10^{-10}$ | $4{,}5.10^{-10}$ | 4,7 | $4{,}22.10^{-10}$ | $6{,}51.10^{-9}$ | 4 |
| avec 5% $CoQ_{10}$ | $2{,}27.10^{-12}$ | $6{,}45.10^{-12}$ | 16,2 | $2{,}8.10^{-11}$ | $2{,}42.10^{-10}$ | 12,7 | $4{,}99.10^{-11}$ | $4{,}56.10^{-10}$ | 11,6 | $6{,}1.10^{-11}$ | $5{,}51.10^{-10}$ | 11,5 |
| sans $CoQ_{10}$ | $1{,}52.10^{-10}$ | $1{,}54.10^{-10}$ | 14,3 | $9.10^{-10}$ | $1{,}61.10^{-8}$ | 0 | - | - | 0 | - | - | 0 |

Légende: - : non déterminé, valeur trop élevée.

**Suite du tableau 1**

| Durée d'entreposage (j) | 16 | | | 17 | | | 20 | | |
|---|---|---|---|---|---|---|---|---|---|
| Echantillon huile de poisson | Penthane M | Ethane M | 02 % | Pentane M | Ethane M | 02 % | Pentane M | Ethane M | 02 % |
| avec 0,1% $CoQ_{10}$ | - | - | 0 | - | - | 0 | - | - | 0 |
| avec 1% $CoQ_{10}$ | $6{,}62.10^{10}$ | $1{,}06.10^{-8}$ | 1,7 | - | - | 0 | - | - | 0 |
| avec 5% $CoQ_{10}$ | $1{,}17.10^{-10}$ | $1{,}05.10^{-9}$ | 9,6 | $1{,}62.10^{-10}$ | $1{,}83.10^{-9}$ | 7,8 | $3{,}76.10^{-10}$ | $5{,}96.10^{-9}$ | 0,6 |
| sans $CoQ_{10}$ (comparaison) | 2,3 | - | 0 | - | - | 0 | - | - | 0 |

Légende: - : non déterminé, valeur trop élevée

Tableau 2

| Echantillon huile de pépins de cassis | Durée d'entreposage (j) 25 Pentane M | 25 Ethane M | 25 O2 % | 27 Pentane M | 27 Ethane M | 27 O2 % | 28 Pentane M | 28 Ethane M | 28 O2 % |
|---|---|---|---|---|---|---|---|---|---|
| avec 0,1% $CoQ_{10}$ | $5,44.10^{-11}$ | $3,51.6^{-11}$ | 13,6 | $4,09.10^{-11}$ | $2,9.10^{-11}$ | 13,6 | $4,76.10^{-9}$ | $2,2.10^{-9}$ | 5,9 |
| avec 1% $CoQ_{10}$ | $1,4.10^{-11}$ | $9,42.10^{-12}$ | 14,4 | $4,6.10^{-10}$ | $2,61.10^{-10}$ | 11,2 | $1,9.10^{-10}$ | $1,14.10^{-10}$ | 12,6 |
| avec 5% $CoQ_{10}$ | x | x | x | $6,55.10^{-11}$ | $4,17.10^{-11}$ | 13,1 | $3,38.10^{-10}$ | $1,76.10^{-10}$ | 11,7 |
| sans $CoQ_{10}$ (comparaison) | $2,71. 10^{-10}$ | $1,64.10^{-10}$ | 12,1 | $1,01.10^{-8}$ | $4,35.10^{-9}$ | 4,6 | $1,31.10^{-8}$ | $5,47. 10^{-9}$ | 2,9 |

Suite du tableau 2

| Echantillon huile de pépins de cassis | Durée d'entreposage (j) 30 Pentane M | 30 Ethane M | 30 O2 % | 31 Pentane M | 31 Ethane M | 31 O2 % | 34 Pentane M | 34 Ethane M | 34 O2 % |
|---|---|---|---|---|---|---|---|---|---|
| avec 0,1% $CoQ_{10}$ | - | - | 0 | - | - | 0 | - | - | 0 |
| avec 1% $CoQ_{10}$ | $3,68.10^{-9}$ | $1,65.10^{-9}$ | 7,1 | $3,11.10^{-8}$ | $1,15.10^{-8}$ | 0 | - | - | 0 |
| avec 5% $CoQ_{10}$ | $8,44.10^{-11}$ | $4,97.10^{-11}$ | 12,8 | $8,38.10^{-10}$ | $4,12.10^{-10}$ | 9 | $5,58.10^{-9}$ | $2,15.10^{-9}$ | 4,4 |
| sans $CoQ_{10}$ | - | - | 0 | - | - | 0 | - | - | 0 |

Légende: - : non déterminé, valeur trop élevée

x : non mesuré

Les résultats précédents montrent clairement l'activité antioxydante de $CoQ_{10}$ dans les huiles de poisson et de pépins de cassis en comparaison avec les mêmes huiles sans $CoQ_{10}$.

Exemple 2

On procède comme à l'exemple 1 à la mesure des teneurs en pentane, ethane et oxygène de l'espace de tête de boîtes de 200 ml contenant 0,5 g d'huile de poisson stabilisée avec:

2.1. 0,1% de $CoQ_{10}$

2.2 0,1% de $CoQ_{10}$ et 1% de lécithine de soja purifiée (Topcithin®, LC)

2.3 0,1% de $CoQ_{10}$, 1% deTopcithin® et 1000 ppm (partie par million) d'acide ascorbique (AA) et entreposés à 37 ° C pendant la durée indiquée (j)

A titre de comparaison, on détermine les teneurs en pentane, éthane et oxygène de l'espace de tête des boîtes contenant l'huile de poisson sans stabilisant ($C_1$), avec 1000 ppm d'AA ($C_2$) et enfin avec 1000 ppm d'AA + 1% de LC ($C_3$).

Les résultats obtenus sont indiqués dans le tableau 3 ci-après:

Tableau 3

| Echantillon huile de poisson | Durée d'entreposage (j) 12 Pentane M | 12 Ethane M | 12 O2 % | 19 Pentane M | 19 Ethane M | 19 O2 % | 41 Pentane M | 41 Ethane M | 41 O2 % |
|---|---|---|---|---|---|---|---|---|---|
| 2.1 | $1,38.10^{-11}$ | $9,77.10^{-11}$ | 13,3 | $4,96.10^{-10}$ | $6,76.10^{-9}$ | 2 | - | - | 0 |
| 2.1 | $1,21.10^{-12}$ | $1,04.10^{-11}$ | 14,6 | $2,01.10^{-11}$ | $2,55.10^{-10}$ | 12,2 | - | - | 0 |
| 2.3 | $5,53.10^{-12}$ | $3,59.10^{-12}$ | 15,4 | $8.10^{-12}$ | $8.10^{-12}$ | 15,4 | $1,73.10^{-10}$ | $3,14.10^{-9}$ | 8 |
| $C_1$ | $2,02.10^{-11}$ | $1,21.10^{-10}$ | 13,1 | $8,56.10^{-10}$ | $1,38.10^{-8}$ | 0 | - | - | 0 |
| $C_2$ | $2.10^{-11}$ | $1,01.10^{-11}$ | 13 | $8.10^{-10}$ | $0,67.10^{-8}$ | 0,5 | - | - | 0 |
| $C_3$ | $1,8.10^{-11}$ | $0,9.10^{-10}$ | 12,9 | $7,8.10^{-10}$ | $0,9.10^{-8}$ | 0,6 | - | - | 0 |

Légende: - : non déterminé, valeur trop élevée

Les résultats précédents montrent clairement que la combinaison de $CoQ_{10}$, d'AA et de LC aux teneurs utilisées procure une protection efficace de l'huile de poisson contre l'oxydation même après 41 j d'entreposage alors que, sans stabilisant ($C_1$), l'huile de poisson s'oxyde rapidement dès le 12ème jour. Par ailleurs l'utilisaion d'AA seul ($C_2$) ou en mélange avec LC ($C_3$) ne permet plus la protection de l'huile de poisson contre l'oxydation à partir du 19ème jour.

## Revendications

**1.** Procédé de protection d'une matière grasse ou d'un produit alimentaire, cosmétique ou pharmaceutique contenant une matière grasse de l'oxydation, caractérisé par le fait que l'on incorpore à la matière

grasse ou au produit alimentaire, cosmétique ou pharmaceutique 0,1 à 5% en poids de coenzyme Q, par rapport au poids de matière grasse.

**2.** Procédé selon la revendication 1, caractérisé par le fait que l'on incorpore à la matière grasse ou au produit alimentaire, cosmétique ou pharmaceutique des quantités efficaces de coenzyme Q, d'acide ascorbique et d'émulsifiant naturel.

**3.** Procédé selon la revendication 2, caractérisé par le fait que l'on incorpore à la matière grasse ou au produit alimentaire, cosmétique ou pharmaceutique un mélange contenant, 2,5 à 10% de coenzyme Q et 2,5 à 20% en poids d'acide ascorbique, par rapport au poids d'émulsifiant naturel, de sorte que la quantité de coenzyme Q est 0,1 à 5% par rapport au poids de la matière grasse.

**4.** Procédé selon la revendication 1, caractérisé par le fait que la coenzyme Q est la coenzyme $Q_{10}$.

**5.** Matière grasse ou produit alimentaire, cosmétique ou pharmaceutique contenant une matière grasse protégé de l'oxydation par la mise en oeuvre du procédé selon l'une des revendications 1 à 5.

**6.** Matière grasse animale, notamment l'huile d'animaux marins protégée de l'oxydation par la mise en oeuvre du procédé selon l'une des revendications 1 à 5.

**7.** Huile végétale, notamment de pépins de cassis protégée de l'oxydation par la mise en oeuvre du procédé selon l'une des revendications 1 à 5.

**8.** Utilisation de la coenzyme Q dans un produit alimentaire, cosmétique ou pharmaceutique contenant une matière grasse pour la protéger de l'oxydation, à raison de 0,1 à 5% en poids de coenzyme Q, par rapport au poids de matière grasse.

**9.** Utilisation de la coenzyme Q en combinaison avec l'acide ascorbique et un émulsifiant naturel dans un produit alimentaire, cosmétique ou pharmaceutique pour le protéger de l'oxydation, à raison de 0,1 à 5% en poids de coenzyme Q, par rapport au poids de matière grasse.

## Claims

**1.** A process for the protection of a fat or a food, cosmetic or pharmaceutical product containing a fat against oxidation, characterized in that coenzyme Q is incorporated in the fat or in the food, cosmetic or pharmaceutical product in a quantity of 0.1 to 5% by weight, based on the weight of the fat.

**2.** A process as claimed in claim 1, characterized in that effective quantities of coenzyme Q, ascorbic acid and natural emulsifier are incorporated in the fat or in the food, cosmetic or pharmaceutical product.

**3.** A process as claimed in claim 2, characterized in that a mixture containing 2.5 to 10% coenzyme Q and 2.5 to 20% by weight ascorbic acid, based on the weight of natural emulsifier, is incorporated in the fat or in the food, cosmetic or pharmaceutical product so that the quantity of coenzyme Q is from 0.1 to 5%, based on the weight of the fat.

**4.** A process as claimed in claim 1, characterized in that the coenzyme Q is the coenzyme $Q_{10.}$

**5.** A fat or a fat-containing food, cosmetic or pharmaceutical product protected against oxidation by application of the process claimed in any one of claims 1 to 4.

**6.** An animal fat, more particularly the oil of marine animals, protected against oxidation by application of the process claimed in any one of claims 1 to 4.

**7.** Vegetable oil, more particularly black currant seed oil, protected against oxidation by application of the process claimed in any one of claims 1 to 4.

**8.** The use of the coenzyme Q in a food, cosmetic or pharmaceutical product containing a fat to protect it against oxidation in a quantity of 0.1 to 5% by weight, based on the weight of the fat.

9. The use of the coenzyme Q in combination with ascorbic acid and a natural emulsifier in a food, cosmetic or pharmaceutical product to protect it against oxidation in a quantity of 0.1 to 5% by weight, based on the weight of the fat.

**Patentansprüche**

1. Verfahren zum Schutz eines Fettes oder eines ein Fett enthaltenden Nahrungsmittels oder kosmetischen oder pharmazeutischen Produkts gegen Oxidation, dadurch gekennzeichnet, daß man in das Fett oder in das Nahrungsmittel oder kosmetische oder pharmazeutische Produkt 0,1 bis 5 Gew.-% Coenzym Q, bezogen auf das Fettgewicht, einbringt.

2. Verfahren nach Anspruch 1, dadurch gekennzeichnet, daß man in das Fett oder in das Nahrungsmittel oder kosmetische oder pharmazeutische Produkt wirksame Mengen von Coenzym Q, Ascorbinsäure und natürlichem Emulgator einbringt.

3. Verfahren nach Anspruch 2, dadurch gekennzeichnet, daß man in das Fett oder das Nahrungsmittel oder das kosmetische oder pharmazeutische Produkt eine Mischung einbringt, die 2,5 bis 10 Gew.-% Coenzym Q und 2,5 bis 20 Gew.-% Ascorbinsäure, bezogen auf das Gewicht des natürlichen Emulgators, enthält, so daß die Menge an Coenzym Q 0,1 bis 5 %, bezogen auf das Gewicht des Fetts, beträgt.

4. Verfahren nach Anspruch 1, dadurch gekennzeichnet, daß das Coenzym Q das Coenzym $Q_{10}$ ist.

5. Fett oder ein Fett enthaltendes Nahrungsmittel oder kosmetisches oder pharmazeutisches Produkt, das durch das Verfahren nach einem der Ansprüche 1 bis 4 gegen Oxidation geschützt ist.

6. Tierisches Fett, insbesondere Öl von Meerestieren, das durch das Verfahren nach einem der Ansprüche 1 bis 4 gegen Oxidation geschützt ist.

7. Pflanzliches Öl insbesondere von Kernen der schwarzen Johannisbeere, das durch das Verfahren nach einem der Ansprüche 1 bis 4 gegen Oxidation geschützt ist.

8. Verwendung des Coenzyms Q in einem Fett enthaltenden Nahrungsmittel oder kosmetischen oder pharmazeutischen Produkt, um dieses Fett gegen Oxidation zu schützen, in einem Anteil von 0,1 bis 5 Gew.-% Coenzym Q, bezogen auf das Fettgewicht.

9. Verwendung des Coenzyms Q in Kombination mit Ascorbinsäure und einem natürlichen Emulgator in einem Nahrungsmittel oder kosmetischen oder pharmazeutischen Produkt zu dessen Schutz gegen Oxidation in einem Anteil von 0,1 bis 5 Gew.-% Coenzym Q, bezogen auf das Fettgewicht.